# EUROPEAN PATENT SPECIFICATION

(11) **EP 3 207 913 B1**
(45) Date of publication and mention of the grant of the patent: **08.01.2020**
(21) Application number: 17155968.5
(22) Date of filing: 14.02.2017
(51) Int. Cl.: A61G 13/12, A61G 13/00, A61F 5/37, A61G 13/10

(54) **ASSEMBLY AND SYSTEM FOR DISTRACTOR AND/OR POSITIONER**
DISTRAKTOR- UND/ODER POSITIONIERUNGSVORRICHTUNG UND -SYSTEM
ENSEMBLE ET SYSTÈME POUR DISTRACTION ET POSITIONNEMENT D'UN MEMBRE LORS D'OPÉRATION CHIRURGICALE

(30) Priority: 19.02.2016 US 201615048577
(43) Date of publication of application: 23.08.2017
(73) Proprietor: Innovative Medical Products, Inc., Plainville, CT 06062 (US)
(72) Inventor: De Mayo, Edward, Plainville, CT 06062 (US); Blackwell, Tim, Plainville, CT 06062 (US); Kovacs, Tamas, Plainville, CT 06062 (US)
(74) Representative: Hentrich Patentanwälte PartG mbB

(56) References cited:
- GB-A- 1 376 386
- US-A- 5 056 535
- US-B1- 8 048 082

## Description

### Cross-Reference To Related Application

This application claims the benefit of U.S. Provisional Application No. 62/118,009 filed Feb. 19, 2016 for "Assembly And System For Distractor And/Or Positioner."

### Field of the Invention

The present invention relates to reducing the tissue trauma that occurs when a physician distends or otherwise deforms a patient's tissues in a surgical Operation or for other medical procedures and, more particularly, to an improved positioning pad assembly and system for securing and holding a limb in a particular position and/or in conjunction with distraction of a joint, bone that advantageously reduces slippage and is particularly useful in the positioning of a larger, heavier body part.

### Background of the Invention

Conventional positioning and distraction systems are useful in positioning a body part (*e.g*. limb, knee, bone, joint, etc.) of a patient for robotic, laparoscopic and other medical procedures when a physician distends or otherwise deforms a patient's tissues in a surgical operation. For example, distraction may be used in medical procedures to separate two parts of a bone after the bone is transected. Also, distraction may be used in surgical procedures to provide distraction of joint surfaces without displacement and/or rupture of their binding ligaments. Consequently, these endoscopic, robotic, laparoscopic and other medical procedures use surgical distraction systems to separate bones, bony fragments or joint surfaces of a limb, *e.g.* when such extension is desired. An conventional positioning system is disclosed in GB 1 376 386 showing an apparatus comprising a knee support located at one end of a table at a level above the latter, and a foot holder located substantially at the level of the knee support. The support is carried by one arm of an angle member, the other arm of which is pivotally connected to two supporting arms. The knee support supports the hollow of the knee with the knee joint forming substantially a right angle.

Conventional surgical positioning and distraction systems may use a foam pad and/or a cohesive wrap to protect a patient from pressure sores, abrasions and possible neurological impairment while securing foot into the boot. For example, sterile foam pads and wraps may be utilized with surgical distraction systems for reducing the pressure placed against the thigh in a medical procedure on a knee or ankle (*e.g*. pressure sores, abrasions and possible neurological impairment). In such medical procedures, a foam pad and/or wrap also must be sterilized and are typically single use.

Conventional sterile foam pads have problems in maintaining position on a bracket and/or in a boot device as these may slip or shift when a distractor is used for flexion, extension, tilt, and rotation, *e.g*. when distracting the ankle or knee during arthroscopic surgery. Such problems in conventional wraps and foam pads are amplified when heavier, larger limbs of a heavier and/or larger patient are being positioned for surgery as well as during surgery. One solution has been to increase the pressure on the pad using distraction; however, pads slide off of the support post, fail and any such increased pressure can be harmful to the circulatory system, tissues, bones, and patient health. As a result, there is a need for an improved positioning pad assembly and system for securing and holding a limb in a particular position and/or in conjunction with distraction of a joint, bone or in the positioning of a body part for a surgical Operation to overcome these and other harmful problems to the circulatory system, tissues, bones, and patient health that are present in conventional surgical positioning and distraction systems.

The present invention solves these problems with a pad assembly and system that has improved holding, resists slippage, and results in less harm to the patient's circulatory system, tissues, and/or bones, thereby improving a patient's health. The improved pad assembly and system according to the present invention maintains its hold and position on a support bar or distractor bar bracket, thereby allowing reduced applied pressure of the distractor against the thigh as compared to conventional surgical positioning and distraction systems. Moreover, the present invention reduces repositioning because its design reduces support bar sliding and slippage especially when distracting heavier, larger limbs of a heavier and/or larger patient before and during a surgical procedure.

### Summary of the Invention

It is an object of the present invention to provide a pad assembly and system of positioning and holding a foam pad assembly that reduces and/or eliminates harmful problems to the circulatory system, tissues, bones, patient health, and otherwise is gentler for the patient.

An object of the present invention is to provide a pad assembly and system for securing and holding a limb in a particular position and/or in conjunction with the positioning of a body part, distraction of a joint, and distraction of bone in a medical procedure.

Another object of the present invention is to provide a protector pad assembly and system for reducing harm to the circulatory system, tissues, bones, and improving patient health by distributing pressure over a large area of the distal femur reducing the direct application of pressure an tissues, vessels and the popliteal nerve.

Yet another object of the present invention is to provide a protector pad assembly and system that maintains its position so as not to shift, slide or roll into the popliteal area. It is an object of the present invention to provide these advantages for a pad assembly that overcomes the shifting into the popliteal area and related problems that are amplified when larger, heavier limbs are being positioned for surgery and throughout a surgical procedure.

Still yet another object of the present invention is to provide a protector pad assembly and system with advantages of a sterilized product that may be produced and sold as a pressure protector pad set or kit for use with a positioning and/or distractor system such as, for example, the De Mayo Universal Distractor as disclosed in U.S. Patent No. 8,048,082 B1.

It is an object of the present invention to provide an apparatus and system of positioning and holding a sterile pad assembly and system with design features for locking to a support bar so as to hold and maintain its position thereby reducing pressure needed to be placed against the thigh in a typical procedure without repositioning.

### Brief Description of the Drawings

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Description of the Embodiments, which is to be read in association with the accompanying drawings, which are incorporated in and constitute a part of this specification, show certain aspects of the subject matter disclosed herein and, together with the description, help explain some of the principles associated with the disclosed implementations, wherein:
FIG. 1 illustrates is a perspective view of the apparatus system in accordance with an embodiment of the present invention;
FIG. 2 illustrates is a side view of the apparatus and system;
**FIG.** 3 illustrates is an end view of the apparatus and system;
FIG. 4 illustrates is a top view of the apparatus and system;
FIG. 5 illustrates is a bottom view of the apparatus and system;
FIG. 6A illustrates a bottom view, FIG. 6B illustrates a side view, and FIG. 6C illustrates a sectional view of the apparatus and system along circle line C-C in accordance with an embodiment of the present invention;
FIG. 7A illustrates a side view of the side portion and opening taken along lines A-A of FIG. 6A; and 7B illustrate is sectional view along circle line B-B of FIG. 7A of the apparatus and system in accordance with an embodiment of the present invention;
FIG. 8 illustrates a perspective view of the foam protector pad;
**FIG.** 9 illustrates an end view of the foam protector pad;
FIG. 10 illustrates a top and or bottom view of the foam protector pad;
FIG. 11 illustrates an end view of the foam protector pad bend to shape in accordance with an embodiment of the present invention;
FIG. 12 illustrates is a perspective view of the foam protector pad bend to shape;
FIG. 13 illustrates is a side view of the foam protector pad bend to shape;
FIG. 14 illustrates is a side view of support of the pad assembly and system in accordance with an embodiment of the present invention
FIG. 15 illustrates is a bottom view of support of the pad assembly and system
FIG. 16 illustrates is an end view of the support of the pad assembly and system;
FIG. 17 illustrates is a side perspective view of the support the pad assembly and system in a surgical Operation with a distractor; and
FIG. 18 illustrates is an anatomical view of the posterior of a human limb.

### Description of the Embodiments

Non-limiting embodiments of the present invention will be described below with reference to the accompanying drawings, wherein like reference numerals represent like elements throughout. While the invention has been described in detail with respect to the preferred embodiments thereof, it will be appreciated that upon reading and understanding of the foregoing, certain variations to the preferred embodiments will become apparent.

The terms "a" or "an", as used herein, are defined as one or as more than one. The term "plurality", as used herein, is defined as two or as more than two. The term "another", as used herein, is defined as at least a second or more. The terms "including" and/or "having", as used herein, are defined as comprising (i.e., open language). The term "coupled", as used herein, is defined as connected, although not necessarily directly, and not necessarily mechanically.

Reference throughout this document to "some embodiments", "one embodiment", "certain embodiments", and "an embodiment" or similar terms means that a particular feature, structure, or characteristic described in connection with the embodiment is included in at least one embodiment of the present invention. Thus, the appearances of such phrases or in various places throughout this specification are not necessarily all referring to the same embodiment. Furthermore, the particular features, structures, or characteristics may be combined in any suitable manner in one or more embodiments without limitation.

The term "or" as used herein is to be interpreted as an inclusive or meaning any one or any combination. Therefore, "A, B or C" means any of the following: "A; B; C; A and **B;** A and C; B and C; A, B and C". An exception to this definition will occur only when a combination of elements, functions, steps or acts are in some way inherently mutually exclusive.

The drawings featured in the figures are provided for the purposes of illustrating some embodiments of the present invention, and are not to be considered as limitation thereto. Term "means" preceding a present participle of an Operation indicates a desired function for which there is one or more embodiments, i.e., one or more methods, devices, or apparatuses for achieving the desired function and that one skilled in the art could select from these or their equivalent in view of the disclosure herein and use of the term "means" is not intended to be limiting.

As used herein the term "popliteal area" refers to the posterior area of the human leg around the knee joint and its tissues, bones circulatory and nerve structures. The area is visually identifiable as a shallow depression located at the back of the knee joint that includes the popliteal artery, popliteal nerve, tibial artery, tibial nerve and other tissues.

As used herein the term "popliteal artery" refers to an artery that is a deeply placed continuation of the femoral artery after it passes through the adductor hiatus, or opening in the distal portion of the adductor magnus muscle. The popliteal artery courses through the popliteal fossa and ends at the lower border of the popliteus muscle, where it branches into the anterior and posterior tibial arteries.

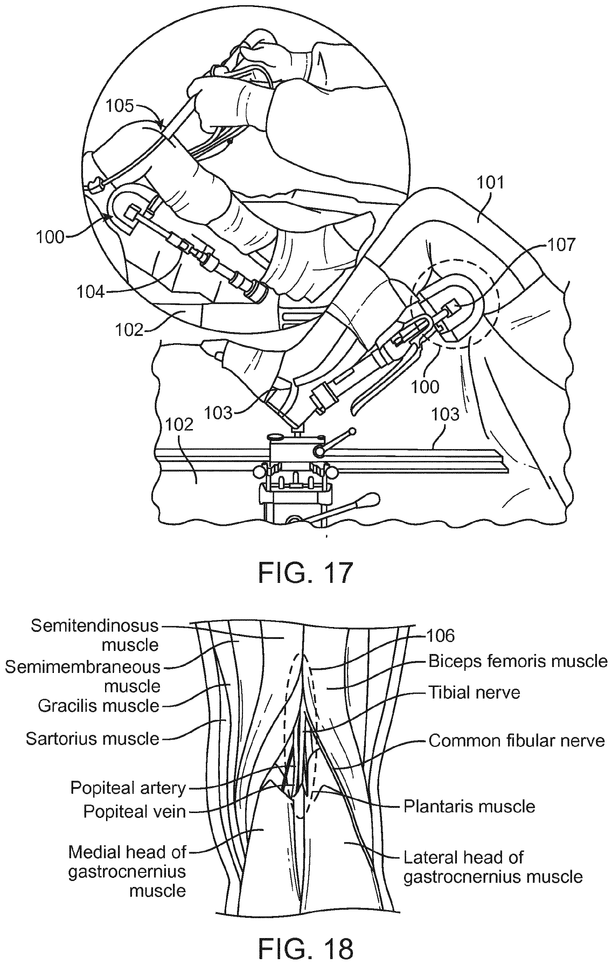

As used herein the term "popliteal nerve" refers to a common fibular nerve (common peroneal nerve; external popliteal nerve; peroneal nerve; lateral popliteal nerve), about one-half the size of the tibial nerve, is derived from the dorsal branches of the fourth and fifth lumbar and the first and second sacral nerves. The popliteal nerve descends obliquely along the lateral side of the popliteal fossa to the head of the fibula, close to the medial margin of the biceps femoris muscle and the common peroneal nerve winds round the head of the fibula as is illustrated in **FIG. 18****.**

As used herein the term "tibial artery" refers to the anterior tibial artery of the leg functioning to carry blood to the anterior compartment of the leg and dorsal surface of the foot, from the popliteal artery, which also is accompanied the anterior tibial vein, along its course.

As used herein the term "tibial nerve" refers to the tibial nerve that is a branch of the sciatic nerve. The tibial nerve passes through the popliteal fossa to pass below the arch of soleus.

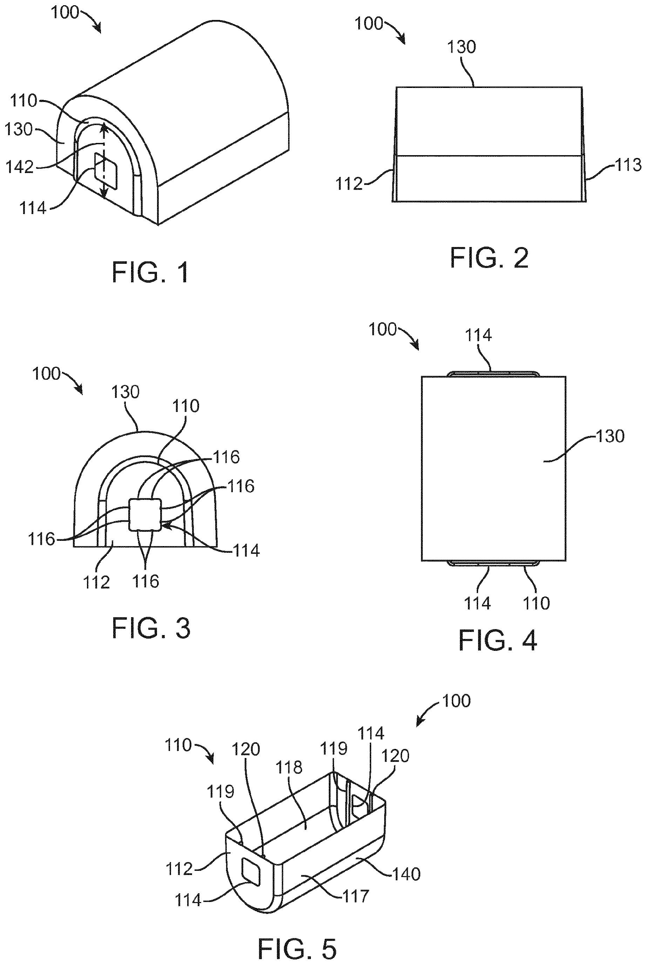

As is illustrated in FIGS. 1-18, the pressure pad apparatus and system 100, such as shown in FIG. 1, is useful to sufficiently optimize secure, hold, and maintain the limb of a patient 101 *(e.g.* leg, knee, ankle, etc.) in a desired position against displacement and pressure forces during a medical procedure to the pressure pad apparatus and system 100 disposed on a support bar of a surgical distractor unit. As shown in FIG. 17, the limb of the patient 101 is set in a desired position on a support or operating (OR) table 102 configured with a positioning system 103, a distractor assembly 104, or both (103 and 104) in a medical procedure 105. Accordingly, the present invention is described in an arthroscopic surgery medical procedure 105 in an embodiment of arthroscopy and/or arthroscopic surgery for the repair of the joint *(i.e.* on the knee as shown in FIGS. 17 and 18) using the pressure pad apparatus and system 100, illustrated in FIGS. 1-16. As shown in FIGS. 17 and 18, the pressure pad apparatus and system is configured to work with a positioner 103 and/or distractor 104 such as the De Mayo Universal Distractor® for the knee and ankle surgical procedures involved in the finite joint distraction of the surgical site, which is described in U.S. Patent No. 8,048,082 B1. Although it should appreciated that the principals of the present invention may be used in other arthroscopic surgery medical procedures commonly used for ankle, hip, wrist, elbow, shoulder problems, whereby the improvements and advantages of the pressure pad apparatus and system 100 may further apply to disadvantages in the prior art for the reduction of injury to the joint, tissues, bones, circulatory and nerve structures.

Referring to FIG. 17, in predetermined arthroscopic surgery on the knee medical procedure 105, the positioning pad and system 100 is attached to the limb support bar of a positioning system 103 that secures and holds the limb of the patient 101 *(i.e.* upper and lower leg and foot) to prevent movement on the OR table 102, which movements could be disruptive to a medical procedure 105 being performed on the patient 101. A pressure pad apparatus 100 may be utilized with the positioning system 103 so as to hyper-extend the joint *(i.e.* knee or ankle in this configuration) for the medical procedure as illustrated in FIG. 17. Simply, a limb of the patient 101 is positioned on the pressure pad apparatus 100 that is disposed on the limb support bar or post 107 of the positioning system 103. The pressure pad apparatus is also connected to the distractor assembly 104 used to hyper-extend the knee for the medical procedure as illustrated in FIG. 17.

According to the knee arthroscopic surgery embodiment of the present invention, the pressure pad apparatus and system 100 has advantages in the reduction of injury to the joint, tissues and arterial structures of the popliteal area 106 around the knee joint and its tissues, bones, circulatory and nerve structures when distracting a knee for a surgical Operation as shown in FIG. 17. In this embodiment, the popliteal area 106 of the patient 101 is protected from injury by the cooperation of the pad assembly 130 with the cage assembly 110. The spring lever of the pad assembly, system and kit 100 is configured to provide a sterile protector pad with a broad distribution of pressure thereby resisting crushing, tearing at the opening 114, or otherwise slipping, sliding, rotating and/or rolling off of the support bar according to an embodiment of the present invention.

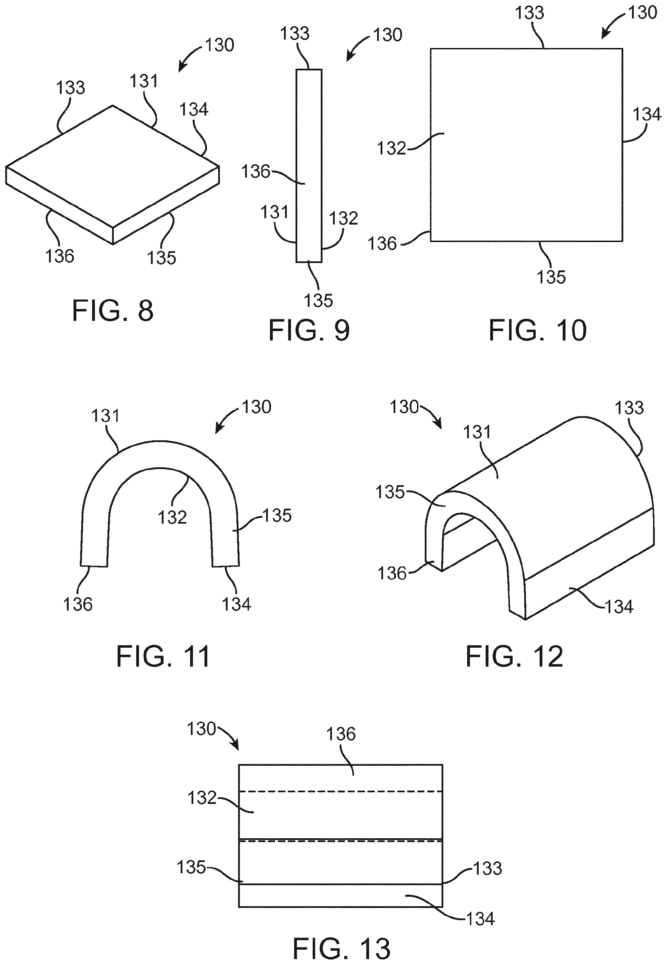

As is illustrated in FIGS. 1-18, the pressure pad apparatus and system 100 comprises of a cage assembly 110 and a pad assembly 130. As is illustrated in FIGS. 8-13, the pad assembly 130 of the pressure pad apparatus and system 100 may be configured as a generally rectangular pad. The pad assembly 130 has a top surface 131, bottom surface 132, and sides 133, 134, 135 and 136. It is to be appreciated that the top surface and the bottom surface may be the same and interchangeable. For ease of the description, the top surface 131 is disposed adjacent the patient's 101 limb near the joint to be extended such as, for example, in the popliteal area 106. The bottom surface 132 the pad assembly 130 is affixed by adhesive to the outer surface 117 on the enclosure 111 of cage assembly 110. The pad assembly 130 may be formed from suitable foam and cell structure materials that can be sterilized such as, for example, polyurethane-based foam. The enclosure or cage assembly 110 is configured with one or more ridges to sufficiently optimize a material strength to the arcuate side of the enclosure so as to not crush under pressure from the surgical distractor unit and/or the weight of the patient's limb of the patient when the sterile cage assembly 110 and pad assembly 130 support a limb of a patient as the support bar of the distractor unit positions the pad assembly 130 in abutment with a popliteal area of a patient's knee joint during a medical procedure.

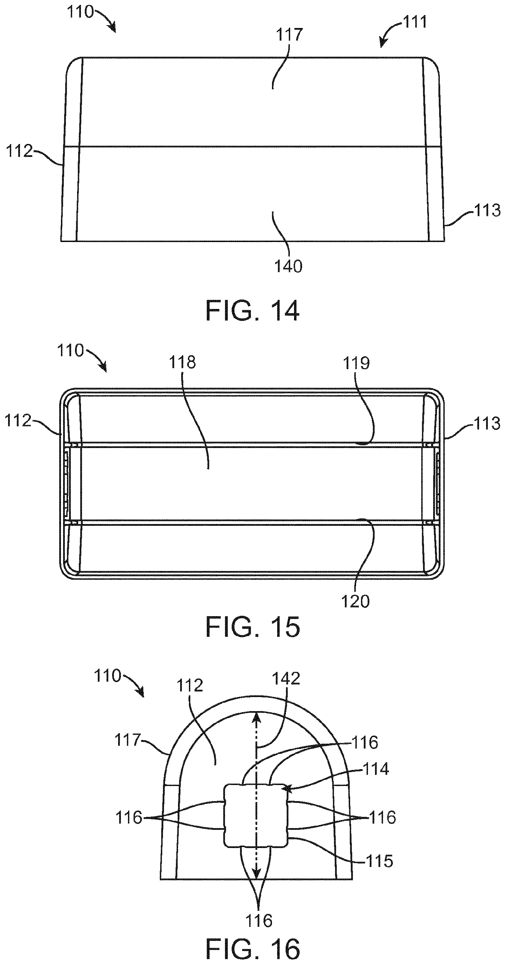

As is illustrated in FIGS. 14-16 the cage assembly 110 comprises an enclosure 111 having a general U-shape and side portions 112 and 113 which may have an opening 114 with a plurality of protrusions or nibs 115 configured to receive the post 107 of a positioning system 103. The enclosure 111 may be formed from plastic materials of suitable strength and durability such as, for example, poly-ester based plastics such as polyethylene terephthalate (PET) and other extruded or molded plastics. The strength of the plastic is required to not crush under pressure from the positioning system 103 such as under the weight of a limb of the patient 101. Moreover, strength of the plastic is required to not tear from pressure from the use of the distractor assembly 104. The durability of the plastic functions to withstand conditions and factors in the operating room, medical procedure and sterilization therefore *(i.e.* high temperatures for proscribed periods of time).

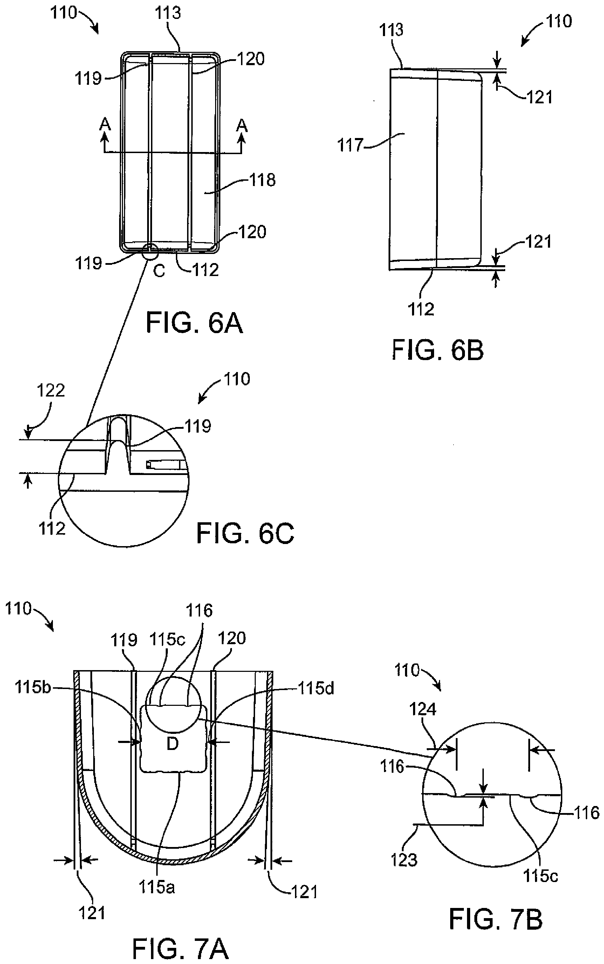

As is illustrated in FIGS. 6A-6C, 7A-7B, 14-16, the enclosure 111 may be formed with side portions 112 and 113 and an arcuate side 140 having an outer surface 117 and an inner surface 118. The outer surface 117 of the arcuate side 140 may be formed generally smooth and in a general U-shape so as to secure the pad assembly 130 thereto such as, for example, using adhesives and the like. The inner surface 118 may be formed with ridges 119 and 120 to provide strength to the arcuate side 140 of the enclosure 111 so as to not crush under pressure from the positioning system 104 such as under the weight of a limb of the patient 101. The one or more ridges 119 and 120 are disposed along the longitudinal axis of the enclosure running along both the side portions 112, 113 and arcuate side 140 (*e.g.* along the inner surface 118). The one or more ridges 119, 120 also are disposed adjacent the opening 114, *i.e.* on either side as illustrated in FIGS. 5, 6A-6C and 7A. The design and placement of the ridges 119 and 120 functions (1) strengthen the area of the arcuate side 140 disposed under the limb of the patient 101 to increase load distribution and resist collapse; and (2) to resist tearing of the opening 114 by the post 107 when stressed by the pressure of the distractor assembly 104 pushing the assembly 100 against the limb of the patient 101 as occurs in the medical procedure 105 *(e.g.* to set and hold the limb in a surgical operation). As shown in FIG. 6C, the one or more ridges 119, 120 are configured to be molded with a predetermined height 122 and shape *e.g*. approximately at an 18 degree spread at the base with a radius of .03. In this manner, the ridges 119 and 120 are configured to reduce weight and cost by using less material for the enclosure 111.

Referring to FIGS. 2 and 6B the side portions 112 and 113 are configured with a predetermined angle 121 may be formed angled (*e.g*. approximately 2 degrees). As illustrated in FIGS. 7A and 7B, the side portions 112, 113 also may have an opening 114 formed therein. As illustrated in FIGS. 6A-6B, 7A-7C, the opening 114 has nibs 116 formed on an inner edge 115 in a predetermined dimension 123 and spaced apart a predetermined distance 124. The opening 114 is dimensioned to receive the post 107 of the positioning system 103. The nibs 116 are formed on inner edge 115 of the opening 114 and further may be designated edges 115a, 115b, 115c, and 115d as illustrated in FIG. 7A.

The side portions 112, 113 are disposed at a predetermined angle 121 of approximately between 1 and 5 degrees measured from an apex of the u-shape of the arcuate side 140 to a lower edge of one or more of the side portions 112, 113. The edges 115a and 115c and the angle of the side portions 112 and 113 (*e.g.* preferably 2 degree displacement angle from apex (top) to side edge (bottom)) cooperate to form a spring lever suitable for locking the assembly to the support bar, *e.g.* of a surgical distraction system. The edges 115b and 115d also provide friction fit on post 107. The spring lever of the edges 115a and 115c uses spaces apart and displaces the nibs 116 on surface 115a and nibs 116 on surface 115c such that when stress is applied by the distractor assembly 104 these nibs 116 create a stronger hold on the support bar or post 107. The spring lever provides a broad distribution of pressure on the protector pad so as not to slip, rotate or roll according to an embodiment of the present invention.

As illustrated in FIGS. 7A and 7B, the nibs 116 are configured to be molded with a predetermined height 122 and shape into the side portions 112, 113. For example, as shown in FIG. 7B, the nibs 116 are formed in a predetermined dimension 123 and spaced apart a predetermined distance 124. The predetermined dimension 123 of the nibs 116 functions to maintain the position of the pressure pad apparatus and system 100 on the support bar or post 107. The nibs 116 improve the friction with the post 107 when the distractor assembly 104 increases the load on the limb of the patient 101, which is an improvement over the prior art. For example, problems of slippage and not holding and maintaining the position on the post 107 occur in prior art systems, for example, when large limbs are operated on in the medical procedure 105.

While certain configurations of structures have been illustrated for the purposes of presenting the basic structures of the present invention, one of ordinary skill in the art will appreciate that other variations are possible. Additional advantages and modifications will readily occur to those skilled in the art.

## Claims

1. An assembly for supporting a limb of a patient during a medical procedure (105) configured to be received by a support bar (107) of a surgical distractor unit (104) for separating anatomical portions of the patient's limb (101) during the medical procedure (105), said assembly comprising:
an enclosure (111) configured with side portions (112, 113), an opening (114) formed in each of said side portions (112, 113) adapted to receive slidably the support bar (107), and an arcuate side (140) connected to said side portions (112, 113) formed in a generally u-shape, said enclosure (111) is configured with one or more ridges (119, 120) adjacent the openings (114) to optimize a spring lever, formed by the edges (115a, 115c) of the opening (114) and an angle (121) measured from an apex of the u-shape of the arcuate side (140) to a lower edge of the side portions (112, 113), and under pressure from the surgical distractor unit (104); and
a pad (130) disposed on said arcuate side (140) of said enclosure (111).

2. The assembly of claim 1, wherein said enclosure (111) and pad (130) are suited to be sterilized for supporting a limb of a patient (101) during a medical procedure (105).

3. The assembly of claim 1, wherein said enclosure (111) is configured with one or more ridges (119, 120) on said arcuate side (140) of said enclosure (111) to reduce weight and strengthen the material strength under pressure from the surgical distractor unit (104) and/or the weight of the patient's limb of the patient (101).

4. The assembly of claim 3, wherein said enclosure (111) is configured with said ridges (119, 120) disposed along a longitudinal axis of said enclosure (111) between said side portions (112, 113) and said arcuate side (140).

5. The assembly of claim 1, wherein said side portions (112, 113) are disposed at said predetermined angle (121) of approximately between 1 and 5 degrees from an apex of the u-shape of said arcuate side (140) to a lower edge of said side portions (112, 113).

6. The assembly of claim 1, wherein said pad (130) is affixed to an outer surface (117) of said enclosure (111) thereto using adhesives.

7. The assembly of claim 1, wherein said pad (130) is formed from suitable foam and cell structure materials that can be sterilized including polyurethane-based foam.

8. The assembly of claim 1, in combination with a system for supporting a limb of a patient (101) during a medical procedure (105), said system comprising:
a surgical distractor unit (104) for separating a pair of adjacent bones within a patient's joint during surgery without insertion within said patient (101) comprising an outer cylinder arranged
between a support bar (107) at one end to be positioned adjacent the patient's limb and a base support
bar at an opposite end thereof for attaching for said surgical distractor unit (104) to a support base, said surgical distractor unit (104) configured to move said support bar (107) relative to said base support bar to control a separation distance between said patient's bones, joints and/or tissues before, during and after surgery thereon;
the cage assembly (110) configured to be received by said support bar (107) so as to abut a patient's joint, said cage assembly (110) comprising:
said openings (114) configured with one or more nibs (116) disposed on an edge (115a, 115c) of said openings (114), said nibs (116) configured to optimize holding of said enclosure (111) on said support bar (107) in a desired position against pressure forces during a medical procedure (105).

9. The system of claim 8, wherein said support bar (107) positions said pad (130) assembly in abutment with a popliteal area (106) of a patient's knee joint.

10. The system of claim 8, wherein said pad (130) affixed to a gel base portion from the group of a staple, stitch, glue, adhesive, or other fastener.

11. The system of claim 8, wherein said base support bar includes means for attachment to an operating table (107) side rail or a patient limb support device.

12. The system of claim 8, wherein said side portions (112, 113) are disposed at a predetermined angle of approximately between 1 and 5 degrees from an apex of the u-shape of said arcuate side (140) to a lower edge of said side portions (112, 113).

13. The system of Claim 8, wherein said sterile pad is formed from suitable foam and cell structure materials that can be sterilized including polyurethane-based foam.

14. The assembly of claim 8, wherein said pad (130) for supporting a limb of a procedure (105), is adapted to be in abutment with a popliteal area (106) of a patient's knee joint in a patient anchoring system comprising:
said cage assembly (110) configured to be received by a support bar (107) so as to abut the patient's Joint.

## Patentansprüche

1. Anordnung zum Unterstützen einer Extremität eines Patienten während eines medizinischen Eingriffs (105),
konfiguriert, um von einer Haltestange (107) einer chirurgischen Distraktoreinheit (104) aufgenommen zu werden, um anatomische Abschnitte der Extremität (101) des Patienten während des medizinischen Verfahrens (105) zu trennen, wobei die Anordnung umfasst:
ein Gehäuse (111), das konfiguriert ist - mit Seitenabschnitten (112, 113), einer Öffnung (114), die in jedem der Seitenabschnitte (112, 113) ausgebildet ist, um die Haltestange (107) gleitend aufzunehmen, und einer bogenförmigen Seite (140), die mit den im Allgemeinen in U-Form ausgebildeten Seitenabschnitten (112, 113) verbunden ist, wobei das Gehäuse (111) mit einer oder mehreren Rippen (120) angrenzend an die Öffnungen (114) ausgebildet ist, um einen Federhebel zu optimieren, der durch die Kanten (115a, 115c) der Öffnung (114) und einen Winkel (121) gebildet wird, der von einem Scheitelpunkt der U-Form der bogenförmigen Seite (140) zu einem unteren Rand der Seitenabschnitte (112, 113) gemessen wird und unter Druck von der chirurgischen Distraktoreinheit (104); und
einem Pad (130), das auf der bogenförmigen Seite (140) des Gehäuses (111) angeordnet ist.

2. Anordnung nach Anspruch 1, wobei das Gehäuse (111) und das Pad (130) geeignet sind, sterilisiert zu werden, um eine Extremität eines Patienten (101) während eines medizinischen Verfahrens (105) zu tragen.

3. Anordnung nach Anspruch 1, wobei das Gehäuse (111) mit einem oder mehreren Rippen (119, 120) auf der genannten bogenförmigen Seite (140) des genannten Gehäuses (111) konfiguriert ist, um das Gewicht zu reduzieren und die Materialfestigkeit unter Druck von der chirurgischen Distraktoreinheit (104) und/oder dem Gewicht der Extremität des Patienten (101) zu verstärken.

4. Anordnung nach Anspruch 3, wobei das genannte Gehäuse (111) mit den genannten Rippen (119, 120) konfiguriert ist, die entlang einer Längsachse des genannten Gehäuses (111) zwischen den genannten Seitenabschnitten (112, 113) und der genannten bogenförmigen Seite (140) angeordnet sind.

5. Anordnung nach Anspruch 1, wobei die genannten Seitenabschnitte (112, 113) unter dem genannten vorbestimmten Winkel (121) von etwa 1 bis 5 Grad von einer Spitze der U-Form der genannten bogenförmigen Seite (140) bis zu einer Unterkante der genannten Seitenabschnitte (112, 113) angeordnet sind.

6. Anordnung nach Anspruch 1, wobei das Pad (130) an einer Außenfläche (117) des Gehäuses (111) unter Verwendung von Klebstoffen befestigt ist.

7. Die Anordnung nach Anspruch 1, worin das Pad (130) geformt ist aus geeignetem Schaum und Zellstrukturmaterialien, die sterilisiert werden können, einschließlich Schaum auf Polyurethanbasis.

8. Anordnung nach Anspruch 1, in Kombination mit einem System zum Tragen einer Extremität eines Patienten (101) während eines medizinischen Verfahrens (105), wobei das System umfasst:
eine chirurgische Distraktoreinheit (104) zum Trennen eines Paares benachbarter Knochen innerhalb des Gelenks eines Patienten während der Operation ohne Einbringung in den Patienten (101), umfassend einen äußeren Zylinder, der angeordnet ist,
zwischen einer Haltestange (107) an einem Ende, das angrenzend an die Extremität des Patienten positioniert werden soll, und einer Basisstützenleiste
an einem gegenüberliegenden Ende davon zum Befestigen der chirurgischen Distraktoreinheit (104) an einer Stützbasis, wobei die chirurgische Distraktoreinheit (104) konfiguriert ist, um die Haltestange (107) relativ zu der Basisstützleiste zu bewegen, um einen Trennungsabstand zu kontrollieren zwischen den Knochen, Gelenken und/oder Geweben des Patienten vor, während und nach der Operation,
die Käfiganordnung (110), die konfiguriert ist, um von der Haltestange (107) aufgenommen zu werden, um an einem Patientengelenk anzuliegen, wobei die Käfiganordnung (110) umfasst:
die genannten Öffnungen (114), die mit einer oder mehreren Federn (116) konfiguriert sind, die an einem Rand (115a, 115c) der Öffnungen (114) angeordnet sind, wobei die Federn (116) konfiguriert sind, um das Halten des Gehäuses (111) auf der Haltestange (107) in einer gewünschten Position gegen Druckkräfte während eines medizinischen Verfahrens (105) zu optimieren.

9. System nach Anspruch 8, wobei die Haltestange (107) das Pad (130) in Anlage mit einem Kniekehlenbereich (106) des Kniegelenks eines Patienten positioniert.

10. System nach Anspruch 8, wobei das Pad (130) an einem Gel-Basisabschnitt befestigt ist, aus der Gruppe einer Heftklammer, eines Stichs, eines Klebers, eines Klebstoffs oder eines anderen Befestigungselements.

11. System nach Anspruch 8, wobei die Basisstützleiste Mittel zur Befestigung eines Operationstisches (107) oder einer Patientenextremitätenstützvorrichtung beinhaltet.

12. System nach Anspruch 8, wobei die Seitenabschnitte (112, 113) in einem vorbestimmten Winkel von etwa 1 bis 5 Grad von einem Scheitelpunkt der U-Form der bogenförmigen Seite (140) zu einer Unterkante der Seitenabschnitte (112, 113) angeordnet sind.

13. System nach Anspruch 8, worin das sterile Pad aus geeignetem Schaumstoff und Zellstrukturmaterialien gebildet ist, die sterilisierbar sind, einschließlich Schaum auf Polyurethanbasis.

14. Anordnung nach Anspruch 8, wobei das Pad (130) zum Tragen einer Extremität in einem Verfahren (105) so ausgebildet ist, dass es an einem Kniekehlenbereich (106) des Kniegelenks eines Patienten in einem Patientenverankerungssystem anliegt, umfassend:
die Käfiganordnung (110), konfiguriert um von einer Haltestange (107) aufgenommen zu werden, um an dem Gelenk des Patienten anzuliegen.

## Revendications

1. Ensemble pour supporter un membre d'un patient pendant une procédure médicale (105) configuré pour être reçu par une barre de support (107) d'une unité de distracteur chirurgical (104) pour séparer des parties anatomiques du membre de patient (101) pendant la procédure médicale (105), ledit ensemble comprenant :
une enceinte (111) configurée avec des parties latérales (112, 113), une ouverture (114) formée dans chacune desdites parties latérales (112, 113) adaptée pour recevoir de manière coulissante la barre de support (107), et un côté arqué (140) connecté auxdites parties latérales (112, 113) formé en une forme globalement en U, ladite enceinte (111) est configurée avec une ou plusieurs saillies (119, 120) adjacentes aux ouvertures (114) pour optimiser un levier à ressort, formé par les bords (115a, 115c) de l'ouverture (114) et un angle (121) mesuré d'un sommet de la forme en U du côté arqué (140) à un bord inférieur des parties latérales (112, 113), et sous la pression de l'unité de distracteur chirurgical (104) ; et
un coussinet (130) disposé sur ledit côté arqué (140) de ladite enceinte (111).

2. Ensemble selon la revendication 1, dans lequel lesdits enceinte (111) et coussinet (130) sont appropriés pour être stérilisés pour supporter un membre d'un patient (101) pendant une procédure médicale (105).

3. Ensemble selon la revendication 1, dans lequel ladite enceinte (111) est configurée avec une ou plusieurs saillies (119, 120) sur ledit côté arqué (140) de ladite enceinte (111) pour réduire le poids et renforcer la résistance du matériau sous la pression de l'unité de distracteur chirurgical (104) et/ou le poids du membre de patient du patient (101).

4. Ensemble selon la revendication 3, dans lequel ladite enceinte (111) est configurée avec lesdites saillies (119, 120) disposées le long d'un axe longitudinal de ladite enceinte (111) entre lesdites parties latérales (112, 113) et ledit côté arqué (140).

5. Ensemble selon la revendication 1, dans lequel lesdites parties latérales (112, 113) sont disposées audit angle prédéterminé (121) d'approximativement entre 1 et 5 degrés d'un sommet de la forme en U dudit côté arqué (140) à un bord inférieur desdites parties latérales (112, 113).

6. Ensemble selon la revendication 1, dans lequel ledit coussinet (130) est fixé à une surface externe (117) de ladite enceinte (111) en utilisant des adhésifs.

7. Ensemble selon la revendication 1, dans lequel ledit coussinet (130) est formé à partir d'une mousse et de matériaux à structure alvéolaire appropriés qui peuvent être stérilisés incluant une mousse à base de polyuréthane.

8. Ensemble selon la revendication 1, en combinaison avec un système pour supporter un membre d'un patient (101) pendant une procédure médicale (105), ledit système comprenant :
une unité de distracteur chirurgical (104) pour séparer une paire d'os adjacents à l'intérieur d'une articulation de patient pendant une chirurgie sans insertion à l'intérieur dudit patient (101) comprenant un cylindre externe agencé entre une barre de support (107) au niveau d'une extrémité devant être positionnée adjacente au membre de patient et une barre de support de base au niveau d'une extrémité opposée de celui-ci pour attacher ladite unité de distracteur chirurgical (104) à une base de support, ladite unité de distracteur chirurgical (104) configurée pour déplacer ladite barre de support (107) par rapport à ladite barre de support de base pour régler une distance de séparation entre les os, articulations et/ou tissus dudit patient avant, pendant et après une chirurgie effectuée sur celui-ci ;
l'ensemble cage (110) configuré pour être reçu par ladite barre de support (107) de façon à venir en butée contre une articulation de patient, ledit ensemble cage (110) comprenant :
lesdites ouvertures (114) configurées avec un ou plusieurs ergots (116) disposés sur un bord (115a, 115c) desdites ouvertures (114), lesdits ergots (116) configurés pour optimiser le maintien de ladite enceinte (111) sur ladite barre de support (107) dans une position souhaitée contre les forces de pression pendant une procédure médicale (105).

9. Système selon la revendication 8, dans lequel ladite barre de support (107) positionne ledit ensemble coussinet (130) en butée sur une région poplitée (106) d'une articulation de genou de patient.

10. Système selon la revendication 8, dans lequel ledit coussinet (130) est fixé à une partie de base en gel du groupe constitué d'une agrafe, d'un point, d'une colle, d'un adhésif, ou d'un autre dispositif de liaison.

11. Système selon la revendication 8, dans lequel ladite barre de support de base inclut un moyen d'attache à un rail latéral d'une table d'opération (107) ou un dispositif de support de membre de patient.

12. Système selon la revendication 8, dans lequel lesdites parties latérales (112, 113) sont disposées à un angle prédéterminé d'approximativement entre 1 et 5 degrés d'un sommet de la forme en U dudit côté arqué (140) à un bord inférieur desdites parties latérales (112, 113).

13. Système selon la revendication 8, dans lequel ledit coussinet stérile est formé à partir d'une mousse et de matériaux à structure alvéolaire appropriés qui peuvent être stérilisés incluant une mousse à base de polyuréthane.

14. Ensemble selon la revendication 8, dans lequel ledit coussinet (130) pour supporter un membre d'une procédure (105), est adapté pour être en butée sur une région poplitée (106) d'une articulation de genou de patient dans un système d'ancrage de patient comprenant :
ledit ensemble cage (110) configuré pour être reçu par une barre de support (107) de façon à venir en butée contre l'articulation du patient.
